# EUROPEAN PATENT APPLICATION

(11) **EP 3 943 078 A1**
(43) Date of publication of application: **26.01.2022**
(21) Application number: 20771974.1
(22) Date of filing: 23.01.2020
(51) Int. Cl.: A61K 31/047, A61K 31/133, A61K 31/191, A61K 31/7004, A61K 45/00, A61P 13/12, A61P 35/00, A61P 43/00, A61K 47/26, A61K 47/55

(54) **DRUG CARRIER UTILIZING PROPERTY OF D-ALLOSE BEING UPTAKEN BY CANCER CELL, DRUG DELIVERY METHOD, AND COMPOSITION FOR TREATING RENAL CELL CARCINOMA**

(30) Priority: 20.03.2019 JP 2019052195
(71) Applicant: National University Corporation Kagawa University, Takamatsu-shi, Kagawa 760-8521 (JP)
(72) Inventor: TAOKA, Rikiya, Kita-gun, Kagawa 761-0793 (JP); KAKEHI, Yoshiyuki, Takamatsu-shi, Kagawa 760-8521 (JP); SUGIMOTO, Mikio, Kita-gun, Kagawa 761-0793 (JP); ZHANG, Xia, Kita-gun, Kagawa 761-0793 (JP); MATSUOKA, Yuki, Kita-gun, Kagawa 761-0793 (JP); IZUMORI, Ken, Kita-gun, Kagawa 761-0795 (JP); YOSHIHARA, Akihide, Kita-gun, Kagawa 761-0795 (JP)
(74) Representative: TBK
(86) International application number: PCT/JP2020/002292
(87) International publication number: WO 2020/189000

(57) **Abstract**

To provide a composition for the treatment of renal cell carcinoma and a composition or a method for enhancing the uptake of a drug into renal cell carcinoma cells. Provided are a D-allose-containing composition for the treatment of renal cell carcinoma, and a D-allose-containing composition or method for enhancing the uptake of a drug into renal cell carcinoma cells. The composition is a pharmaceutical composition to be administered in an effective amount to patients requiring the treatment of renal cell carcinoma in humans or animals other than humans.

## Description

### TECHNICAL FIELD

The present invention relates to a selective drug-delivery carrier to renal cell carcinoma cells, a drug delivery method, and a composition for the treatment of renal cell carcinoma, each utilizing the property of D-allose being taken up by carcinoma cells.

### BACKGROUND ART

Cancer treatment methods are roughly classified into surgery therapy, radiation therapy, and chemotherapy. Of these, chemotherapy is a treatment of administering an anticancer agent to cancer patients and is used for the preoperative/postoperative adjuvant chemotherapy for improving healing ability before or after surgery or radiation treatment or the treatment of metastatic cancers that cannot be treated by the surgery or radiation treatment. Although some cancers have reached the point where their cure can be expected as a result of clinical commercialization of anticancer agents such as antimetabolites, topoisomerase inhibitors, molecular target drugs, and nucleic acid-based drugs, no satisfactory results have been achieved in the chemotherapy of renal cell carcinoma.

Anticancer agents often do not selectively act on tumor cells but act on normal cells and cause high toxicity and side effects. The tissue having a high cell division rate is particularly likely to be affected by them and this harmful systemic toxicity limits the dosage of them allowed to administer cancer patients and limits their effects. In addition, some of anticancer agents have low solubility, are hydrophobic, and therefore have low membrane permeability and in addition, they form their agglutinate by remaining without being dissolved in an aqueous medium so that at the time of intravenous administration, they may cause embolization of capillary vessels before they penetrate into tumors.

To selectively deliver a drug to tumors, there has conventionally been developed a drug delivery system, for example, a system of encapsulating a drug in a carrier such as micelles, liposomes, nanoparticles, and antibody- or drug-polymer conjugates (Patent Document 1). In spite of such an attempt to improve the ability for accumulating an anticancer agent to a tumor tissue, it has still been difficult to prevent the anticancer agent from accumulating on the normal tissue of the liver, kidney, or the like. There is therefore a demand for the development of a drug carrier capable of selectively delivering an anticancer agent to carcinoma cells.

Among the results of an applied research of rare sugars in the medical fields, there is an invention of an in vivo antioxidant having D-allose as an effective ingredient (Patent Document 2). The invention relates to the use of D-allose as a composition to be administered to patients of liver cancer or skin cancer for treating their liver cancer or skin cancer by making use of the in vivo antioxidant action of D-allose.

### Prior Art Documents

### Patent Documents

Patent Document 1: Japanese Laid-Open Patent Publication No. 2015-155392
Patent Document 2: Japanese Patent No. 5330976
Patent Document 3: Japanese Laid-Open Patent Publication No. 2004-298106

### Non-Patent Document

Non-Patent Document 1: J. Ferment. Bioeng. 84, 319, 1997

### SUMMARY

### Technical Problem

The present invention is to provide a novel use of D-allose that makes use of its property of being taken up into carcinoma cells, more specifically to provide a novel use, as a drug delivery carrier, of D-allose having a property of being taken up into carcinoma cells. In addition, the present invention is to provide a drug delivery method using the drug delivery carrier. Further, an object of the present invention is to provide, as an anticancer agent, a composition for the treatment of renal cell carcinoma having an excellent antitumor effect on renal cell carcinoma. Another object of the present invention is to provide a composition for the treatment of renal cell carcinoma capable of enhancing the uptake of a drug into renal cell carcinoma cells and a composition or method for enhancing the uptake of a drug into renal cell carcinoma cells.

### Solution to Problem

The present inventors have carried out an extensive investigation to achieve the above-described objects. As a result, it has been found for the first time that D-allose is taken up specifically into human renal cell carcinoma cells and the D-allose thus taken up suppresses the growth of the renal cell carcinoma cells and thus has anti-tumor activity, leading to the completion of the present invention. In addition, the use of D-allose, which is taken up into human renal cell carcinoma cells, as a carrier for a chemotherapeutic or nucleic acid-based drug serving as an anticancer agent makes it possible to enhance the uptake of the anticancer agent into the renal cell carcinoma cells and allow both the D-allose and the anticancer agent thus taken up to exhibit their antitumor activity.

The aspect of the present invention is an agent making use of the property of being taken up selectively into renal cell carcinoma cells according to the following (1) to (4).
(1) An agent characterized by having D-allose and making use of a property of being taken up selectively into renal cell carcinoma cells.
(2) The agent according to the aspect (1), wherein D-allose taken up into renal cell carcinoma cells exhibits an antitumor effect.
(3) The agent according to the aspect (1) or (2), wherein D-allose is D-allose and/or a derivative thereof, and/or a mixture thereof.
(4) The agent according to the aspect (3), wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.

The aspect of the present invention is a selective drug delivery carrier to renal cell carcinoma cells according to the following (5) to (11).
(5) A selective drug delivery carrier to renal cell carcinoma cells, characterized by having D-allose.
(6) The selective drug delivery carrier to renal cell carcinoma cells according to the aspect (5), wherein D-allose taken up into renal cell carcinoma cells exhibits an antitumor effect.
(7) The selective drug delivery carrier to renal cell carcinoma cells according to the aspect (4) or (5), wherein D-allose is D-allose and/or a derivative thereof, and/or a mixture thereof.
(8) The selective drug delivery carrier to renal cell carcinoma cells according to the aspect (7), wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.
(9) The selective drug delivery carrier to renal cell carcinoma cells according to any one of the aspects (5) to (8), wherein the drug contains an anticancer agent.
(10) The selective drug delivery carrier to renal cell carcinoma cells according to any one of the aspects (5) to (9), wherein D-allose and the drug are associated through covalent bonding directly or via a linker.
(11) The selective drug delivery carrier to renal cell carcinoma cells according to any one of the aspects (5) to (10), wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

The aspect of the present invention is a composition for the treatment of renal cell carcinoma according to the following (12) to (19).
(12) A composition for the treatment of renal cell carcinoma, characterized by having D-allose.
(13) The composition for the treatment of renal cell carcinoma according to the aspect (12), wherein D-allose taken up into renal cell carcinoma cells exhibits an antitumor effect.
(14) The composition for the treatment of renal cell carcinoma according to the aspect (12) or (13), wherein D-allose is D-allose and/or a derivative thereof, and/or a mixture thereof.
(15) The composition for the treatment of renal cell carcinoma according to the aspect (14), wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.
(16) The composition for the treatment of renal cell carcinoma according to any one of the aspects (12) to (15), further containing a drug associated with D-allose.
(17) The composition for the treatment of renal cell carcinoma according to the aspect (16), wherein the drug contains an anticancer agent.
(18) The composition for the treatment of renal cell carcinoma according to the aspect (16) or (17), wherein D-allose and the drug are associated through covalent bonding directly or via a linker.
(19) The composition for the treatment of renal cell carcinoma according to any one of the aspects (16) to (18), wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

The aspect of the present invention is a drug delivery method to renal cell carcinoma cells according to the following (20) to (26).
(20) A drug delivery method to renal cell carcinoma cells which is characterized by supporting a drug on a drug delivery carrier to obtain a medicament, using the medicament for renal cell carcinoma cells, allowing the medicament to be taken up selectively into the renal cell carcinoma cells, and thereby delivering the drug selectively to the cells, wherein the carrier is D-allose and the medicament is obtained by supporting the drug on the D-allose through chemical bonding.
(21) The drug delivery method according to the aspect (20), which is a method making use of a property of D-allose being taken up into carcinoma cells and enhancing the uptake of the drug into the renal cell carcinoma cells.
(22) The drug delivery method according to the aspect (20) or (21), wherein the carrier is D-allose and the medicament is obtained by supporting the drug on the D-allose by associating therewith through covalent bonding directly or via a linker.
(23) The drug delivery method according to any one of the aspects (20) to (22), wherein the drug contains an anticancer agent.
(24) The drug delivery method according to any one of the aspects (20) to (23), wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.
(25) The drug delivery method according to any one of the aspects (20) to (24), wherein D-allose is D-allose and/or a derivative thereof, and/or a mixture thereof.
(26) The drug delivery method according to the aspect (25), wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.

### Advantageous Effects of Invention

D-allose is known to have an antitumor effect on liver cancer and skin cancer due to its in vivo antioxidant action, but is not known to have an antitumor effect on renal cell carcinoma. In addition, it has not been proven that D-allose is taken up into human carcinoma cells.

The present inventors have found for the first time that D-allose is taken into human renal cell carcinoma cells and D-allose taken up into the cells has antitumor activity. D-allose is water soluble so that it has high membrane permeability and further, it does not form an aggregate because it is soluble in an aqueous medium.

It is taken up specifically into renal cell carcinoma cells particularly among human carcinoma cells so that it can be used as a drug carrier (selective drug delivery carrier) capable of delivering an anticancer agent selectively to renal cell carcinoma cells, enhancing the uptake of the anticancer agent into renal cell carcinoma cells, and allowing both the D-allose and anticancer agent thus taken up into the cells to exhibit antitumor activity.

The five-year survival rate from the renal cell carcinoma in the clinical stage IV is still as low as 18.1% in Japan so that the D-allose-containing composition for the treatment of renal cell carcinoma according to the present invention has the potential to be a breakthrough molecular targeted remedy.

### BRIEF DESCRIPTION OF DRAWINGS

Fig. 1 shows the results of the viability assay of renal cell carcinoma cell strain (ACHN).
Fig. 2 shows the results of viability assay of renal cell carcinoma cell line (Caki-I).
Fig. 3 shows the results of viability assay of renal cell carcinoma cell line (Caki-II).
Fig. 4 is a graph showing the change of a D-allose concentration in tumor after intraperitoneal infusion into a xenograft mouse model of renal cell carcinoma.
Fig. 5 is a graph showing the change of a tumor volume in a xenograft mouse model of renal cell carcinoma caused by the intraperitoneal injection of D-allose.
Fig. 6 is a graph showing the influence of D-allulose, that is, the change of the body weight in a xenograft mouse model of renal cell carcinoma.
Fig. 7 is a micrograph (× 100) showing the influence of D-allose, that is, the change of the kidney tissue in a xenograft mouse model of renal cell carcinoma.
Fig. 8 is a micrograph (× 100) showing the influence of D-allose, that is, the change of the liver tissue in a xenograft mouse model of renal cell carcinoma.

### DESCRIPTION OF EMBODIMENTS

D-allose is used as an agent making use of its property of being selectively taken up into renal cell carcinoma cells. D-allose taken up into renal cell carcinoma cells exhibits an antitumor effect. In addition, D-allose is used as a selective drug delivery carrier to renal cell carcinoma cells and therefore provides a drug delivery method to renal cell carcinoma cells. The drug contains an anticancer agent and the drug is associated with D-allose by covalent bonding directly or via a linker. The present invention also provides a D-allose-containing composition for the treatment of renal cell carcinoma and it includes a preparation containing an effective amount of D-allose or a pharmacologically acceptable salt or/and a hydrate thereof. In the above drug delivery method, a carrier composed of D-allose is used and as a drug to be supported by the carrier, various ones as described below can be used. The drug can be selected as needed, depending on the intended use. Since the drug supported on the carrier is taken up selectively into renal cell carcinoma cells, the drug is preferably an anticancer agent or the like. This means that for the purpose of the uptake of the carrier composed of D-allose into carcinoma cells, it is desired to prepare a medicament having an anticancer agent supported thereon.

D-allose to be used in the present invention is a rare sugar present in an overwhelmingly small amount in nature compared with D-glucose abundant therein. Of the monosaccharides ((monosaccharides with 6 carbon atoms: hexoses), 34 kinds in total, including 16 kinds of aldoses, 8 kinds of ketoses, and 10 kinds of sugar alcohols), monosaccharides (aldose and ketose) and derivatives thereof present rarely in nature are defined as "rare sugars" as contrasted with "natural monosaccharides" typified by D-glucose present in a large amount in nature. The rare sugars that can be produced much at this time are D-allulose (D-psicose) and D-allose. D-allose is a D form of allose classified into aldose which is a hexose.

D-allose can be obtained by a method of using L-rhamnose isomerase (Non-Patent Document 1) isolated from Pseudomonas stutzeri and synthesizing from D-allulose, a method of allowing D-xylose isomerase to act on a D-allulose-containing solution, or the like. High-purity D-allose is prepared by a fractionating method using the crystallization method of D-allose (Patent Document 3). The D-allose in the present invention is not limited to that obtained by the above method but also that obtained by isomerization by a chemical treatment method. Thus, that obtained by any method can be used. At this time, it is the common practice to produce D-allulose, a raw material of D-allose, by subjecting fructose to an enzymatic (epimerase) treatment. Not only it but also that obtained by a preparation process making use of a microorganism that produces the enzyme or that extracted from a natural material may be used. In addition, that contained in a natural material may be used as is or that isomerized by a chemical treatment method may also be used. A method of purifying D-allulose by making use of an enzyme is known.

The derivative of D-allose will next be described. A compound obtained by converting the molecular structure of a certain starting compound by a chemical reaction is called "a derivative of the starting compound". Examples of common derivatives of hexoses including D-allose include, but not limited to, sugar alcohols (when a monosaccharide is reduced, the aldehyde group or ketone group becomes an alcohol group, and the monosaccharide becomes a polyhydric alcohol having the same number of carbon atoms), uronic acids (obtained by oxidizing the alcohol group of a monosaccharide and D-glucuronic acid, galacturonic acid, and mannuronic acid are known in nature), and amino acids (obtained by substituting the OH group of the monosaccharide molecule by an NH₂ group and examples include glucosamine, chondrosamine, and glucoside). The derivatives of D-allose are D-allose derivatives selected from sugar alcohols obtained by the conversion the carbonyl group of D-allose to an alcohol group, uronic acids obtained by oxidizing the alcohol group of D-allose, and amino acids obtained by substituting the alcohol group of D-allose by an NH₂ group.

The treatment composition containing D-allose and/or a derivative thereof, and/or a mixture thereof according to the present invention contains therein an effective amount of D-allose and/or a derivative thereof, and/or a mixture thereof. The term "effective amount" means any amount enough for satisfying the intended object (for example, a biological or medical response desirable in the tissue or test subject). In the present invention, for example, the object is to suppress the growth of renal cell carcinoma cells or the delivery of a drug into renal cell carcinoma cells.

Next, a preparation of D-allose or a pharmaceutically acceptable salt and/or a hydrate thereof will be described.

A preparation can be obtained by using D-allose, and/or a derivative thereof, and/or a mixture thereof alone or by adding an appropriate additive such as a common excipient, stabilizer, preservative, binder, disintegrant, and the like and using a suitable dosage form selected from liquids, capsules, granules, pills, powders, tablets, and the like. The composition for the treatment of renal cell carcinoma according to the present invention can be produced by a known production technology in various forms containing the active ingredient and pharmaceutically acceptable additives such as carrier, excipient, lubricant, and binder, for example, liquids dissolved in water or various transfusion preparations, powders, granules, tablets, injections, suppositories, or external preparations. As a parenteral agent, a dosage form such as injection, drop, medicament for external use, or suppository can be selected. The injection includes subcutaneous injection, intramuscular injection, intraperitoneal injection, or the like. The technology of producing the preparation in the above dosage form is known.

When D-allose, the pharmaceutically acceptable salt thereof, or the like according to the present invention is administered by injection, an aqueous injection, an injectable aqueous suspension, a lipid emulsion, a liposome injection, and the like are preferred. In the aqueous injection or injectable aqueous suspension, the aqueous injection or injectable aqueous suspension is obtained by mixing the rare sugar D-allose or pharmaceutically acceptable salt thereof according to the present invention with purified water, adding a water-soluble or water-swelling polymer, a pH regulator, a surfactant, an osmotic pressure regulator, an antiseptic, a preservative, or the like if necessary, mixing, dissolving or suspending the resulting mixture while heating if necessary, sterilizing, filling an injection container with the resulting mixture, and hermetically sealing the container. The aqueous injection can be administered intravenously, subcutaneously, intramuscularly, intradermally, in joint cavity, or the like. The injectable aqueous suspension can be administered subcutaneously, intramuscularly, intradermally, in joint cavity, or the like. It can also be administered orally.

Preferred examples of the water-soluble or water-swelling polymer include gelatins, cellulose derivatives, acrylic acid derivatives, povidone, macrogol, polyamino acid derivatives, and polysaccharides. As the gelatins, purified gelatin is preferred. As the cellulose derivatives, preferred are methyl cellulose, hydroxypropylmethyl cellulose 2910, hydroxypropylmethyl cellulose 2208, hydroxypropylmethyl cellulose 2906, hydroxypropyl cellulose, lower substituted hydroxypropyl cellulose, and carmellose sodium. As the acrylic acid derivatives, preferred are aminoacryl methacrylate copolymers and methacrylic acid copolymers; and as the polyamino acid derivatives, preferred are polylysine and polyglutamic acid. As the polysaccharides, hyaluronic acid, dextran, and dextrin are particularly preferred. The amount of the water-soluble or water-swelling polymer to be added varies depending on the properties and amount of D-allose, derivatives thereof, or pharmaceutically acceptable salts thereof, as well as the properties, molecular weight, and applied site of the water-soluble or water-swelling polymer. It may generally be used in the range of 0.01% to 10% of the total amount of the preparation.

As the pH regulator, an acid or alkali harmless to the human body is used; and as the surfactant, a non-ionic surfactant, an anionic surfactant, or an amphoteric surfactant is used. Examples of the osmotic pressure regulator include sodium chloride and glucose, those of the antiseptic include parabens, and those of the preservative include ascorbic acid and sulfites. Although the using amount of them is not particularly limited, they can be used within the ranges in which they can exhibit their actions, respectively. In addition, a local anesthetic such as procaine hydrochloride, a soothing agent such as benzyl alcohol, a chelating agent, a buffer, or a water-soluble organic solvent may be added.

The lipid emulsion may be prepared by blending an emulsifier and D-allose or a pharmacologically acceptable salt thereof in an appropriate fat or oil, adding purified water thereto, adding a water-soluble or water-swelling polymer, pH regulator, surfactant, osmotic pressure regulator, antiseptic, preservative, or the like if necessary, emulsifying the resulting mixture with an appropriate emulsifying apparatus, sterilizing the resulting emulsion, filling an injection container therewith, and hermetically sealing the container.

The "drug" or "anticancer agent" of the present invention is administered to the cancer or precancerous tissue for the treatment and examples include radioisotopes (for example, iodine-131, lutetium-177, rhenium-188, and yttrium-90"), toxins (for example, diphtheria, Pseudomonas, ricin, and gelonin), enzymes, enzymes activating a prodrug, radiosensitizers, interfering RNAs, superantigens, anti-angiogenic agents, alkylating agents, purine antagonists, pyrimidine antagonists, plant alkaloids, intercalating antibiotics, aromatase inhibitors, antimetabolites, mitotic inhibitors, growth factor inhibitors, cell cycle inhibitors, topoisomerase inhibitors, biological response modifiers, antihormones, and antiandrogens..

The drug associated with (for example, bound to or interacted with) D-allose is used. This association may be through covalent bonding or non-covalent bonding. In the association of D-allose with the drug, strength enough for not causing dissociation before or during the delivery to renal cell carcinoma cells or uptake into renal cell carcinoma cells is required so that any chemical, biochemical, or enzymatic coupling known to those skilled in the art can be used.

When the association of D-allose with the drug is through noncovalent bonding, it may be achieved by a hydrophobic interaction, an electrostatic interaction, a dipolar interaction, a Van der Waals interaction, or hydrogen bonding. When the association of D-allose with the drug is through covalent bonding, they may be bound to each other directly or indirectly via a linker. Such covalent bonding can be achieved through amide, ester, carbon-carbon, disulfide, carbamate, ether, thioether, urea, amine, or carbonate bonding.

With respect to the verification of the safety of D-allose necessary for the use of it as a pharmaceutical ingredient, it was expectable that it was safe because a rare sugar was a monosaccharide present in nature, though its amount was trace. A mutagenicity test, a biodegradability test, and three acute toxicity tests (oral acute toxicity test, skin primary irritation test, and eye primary irritation test) have been determined as the most fundamental safety tests. The present inventors asked a designated agency to conduct the fundamental safety tests of D-allose. As a result, it has been confirmed to have no safety problems.

The subjects of the treatment composition of the present invention are animals including humans (mammals such as humans, cows, pigs, dogs, and cats and birds such as chicken). Carcinoma cells which are targets of the treatment composition of the present invention are renal cell carcinoma cells and examples of a cell line include ACHN, Caki-I, and Caki-II which are human renal cell carcinoma cell lines.

The present invention will hereinafter be described in detail by Examples. The present invention is however not limited to or by these Examples.

### Example 1

### [Rare sugar uptake experiment using human bladder carcinoma cell line, human prostatic carcinoma cell line, and human kidney cell carcinoma cell line]

An uptake amount of a rare sugar, which had been added to a medium, into carcinoma cells was analyzed using the cell lines of three types of cancers. As the rare sugar, four kinds, that is, D-allose, D-allulose (D-psicose), L-allulose (L-psicose), and allitol were used, while D-glucose and fructose were used as a monosaccharide which was not a rare sugar.

Cells of each of the cell lines were cultured in RPMI-1640 media and during culturing, six sugars were each added to the resulting media and cultured. After culturing, an amount of each sugar taken up into the cells was analyzed. The medium to which no sugar was added during culturing was used as a control.

### 1) Cell lines used

Used were human bladder carcinoma cell lines (RT112, 253J, and J82), human prostatic carcinoma cell lines (LNCa, Du145, and PC-3), and human renal cell carcinoma cell lines (ACHN, Caki-I, and Caki-II).

### 2) Media used

After cells were cultured in RPMI-1640 (2000 mg D-glucose/L) media, the above monosaccharides and rare sugars were added to the resulting media, respectively. Described specifically, they were following seven combinations: RPM1-1640 alone (control), RPMI-1640 + D-glucose, RPMI-1640 + L-allulose, RPMI-1640 + D-allulose, RPMI-1640 + D-fructose, RPMI-1640 + D-allose, and RPMI-1640 + D-allitol. The final concentration of the sugar in the medium was adjusted to 10 mM and no sugar was added in the control.

### 3) Culturing method

Five-ml portions of a 3.0 × 10⁴ cells/ml cell suspension prepared using RPMI-1640 were seeded in dishes having a diameter of 6 cm (1.5 × 10⁵ cells/dish) and cultured for 24 hours in the RPMI-1640 medium containing 2000 mg/l of D-glucose as a nutrient source. Twenty-four hours later, the media were changed to the culture solutions containing the monosaccharides and rare sugars described above in 2), respectively, followed by culturing for 48 hours.

### 4) Method of analyzing sugar in cultured cells

After culturing for 48 hours, the attached human carcinoma cells were mechanically separated and collected in a Spitz tube together with the culture solution and floating cells. After the supernatant formed by centrifugation (4°C, 1200 rpm, and 5 minutes) was removed to obtain cell pellets alone, 5 ml of a phosphate buffer physiological saline (PBS) was added for the purpose of washing to obtain a cell suspension of the cell pellets. Again, the resulting cell suspension was centrifuged at 4°C and 1200 rpm for 5 minutes and the supernatant was removed. To the cell pellets after washing was added 1 ml of pure water to suspend them therein. Ultrasonic disruption (intensity: 40%, 30 seconds) was then performed and the monosaccharide in the disrupted cell suspension was labeled with ABEE, followed by analysis by HPLC. From the area thus obtained, the sugar content in the cells was determined using a calibration curve. With respect to the ketose, two peaks were found after labeling with ABEE so that the sugar content in the cells was determined from the calibration curves of both peaks.

The results are shown in the following Table 1 (a sugar content in the disrupted cell suspension of human bladder carcinoma cell line RT112), Table 2 (a sugar content in the disrupted cell suspension of human bladder carcinoma cell line 253J), Table 3 (a sugar content in the disrupted cell suspension of human bladder carcinoma cell line J82), Table 4 (a sugar content in the disrupted cell suspension of human prostate carcinoma cell line LNCa), Table 5 (a sugar content in the disrupted cell suspension of human prostate carcinoma cell line Du145), Table 6 (a sugar content in the disrupted cell suspension of human prostate carcinoma cell line PC-3), Table 7 (a sugar content in the disrupted cell suspension of human renal cell carcinoma cell line ACHN), Table 8 (a sugar content in the disrupted cell suspension of human renal cell carcinoma cell line Caki-I), and Table 9 (a sugar content in the disrupted cell suspension of human renal cell carcinoma cell line Caki-II).

**[Table 1]**

| Medium | Sugar | Content in cell (µg) | Sugar | Content in cell (µg) |
|---|---|---|---|---|
| RPM I-1640 | D-glucose | 3.6 | | |
| RPM I-1640 + D-glucose | D-glucose | 16.7 | | |
| RPM I-1640 + L-allulose | D-glucose | 3.9 | L-allulose | 13.8 |
| RPM I-1640 + D-allulose | D-glucose | 3.4 | D-allulose | 10.0-13.0 |
| RPM I-1640 + D-fructose | D-glucose | 4.4 | D-fructose | 12.7-15.7 |
| RPM I-1640 + D-allose | D-glucose | 9.7 | D-allose | 0.0 |
| RPM I-1640 + allitol | D-glucose | 3.6 | allitol | Undetectable |

| | | | | |
|---|---|---|---|---|
| Sugar content in disrupted cell suspension of RT112 | | | | |

As a result of the analysis of the disrupted cell suspension of the bladder carcinoma cell line RT112 by HPLC, the presence of D-glucose in the cells was found in all the media and its content was high in the cells cultured in the media RPMI-1640 + D-glucose and RPMI-1640 + D-allose, each having a high D-glucose content.

The cells cultured in RPMI-1640 + L-allulose contained 13.8 µg of L-allulose; the cells cultured in RPMI-1640 + D-allulose contained 10.0 to 13.0 µg of D-allulose; and the cells cultured in RPMI-1640 + D-fructose contained 12.7 to 15.7 µg of D-fructose.

**[Table 2]**

| Medium | Sugar | Content in cell (µg) | Sugar | Content in cell (µg) |
|---|---|---|---|---|
| RPM I-1640 | D-glucose | 0.9 | | |
| RPM I-1640 + D-glucose | D-glucose | 4.0 | | |
| RPM I-1640 + L-allulose | D-glucose | 0.5 | L-allulose | 15.5-17.0 |
| RPM I-1640 + D-allulose | D-glucose | 0.6 | D-allulose | 14.1-15.8 |
| RPM I-1640 + D-fructose | D-glucose | 3.3 | D-fructose | 0.0 |
| RPM I-1640 + D-allose | D-glucose | 11.5 | D-allose | 0.0 |
| RPM I-1640 + allitol | D-glucose | 0.5 | allitol | Undetectable |

| | | | | |
|---|---|---|---|---|
| Sugar content in disrupted cell suspension of 253J | | | | |

As a result of the analysis of the disrupted cell suspension of the bladder carcinoma cell line 253J by HPLC, the presence of D-glucose was found in the cells in all the media and its content was high in the cells cultured in the media RPMI-1640 + D-glucose, RPMI-1640 + D-fructose, and RPMI-1640 + D-allose, each having a high D-glucose content.

The cells cultured in RPMI-1640 + L-allulose contained 15.5 to 17.0 µg of L-allulose; and the cells cultured in RPMI-1640 + D-allulose contained 14.1 to 15.8 µg of D-allulose.

**[Table 3]**

| Medium | Sugar | Content in cell (µg) | Sugar | Content in cell (µg) |
|---|---|---|---|---|
| RPM I-1640 | D-glucose | 2.5 | | |
| RPM I-1640 + D-glucose | D-glucose | 7.2 | | |
| RPM I-1640 + L-allulose | D-glucose | 1.9 | L-allulose | 13.2-14.1 |
| RPM I-1640 + D-allulose | D-glucose | 2.1 | D-allulose | 11.8-14.3 |
| RPM I-1640 + D-fructose | D-glucose | 2.7 | D-fructose | 11.1-15.0 |
| RPM I-1640 + D-allose | D-glucose | 11.2 | D-allose | 0.0 |
| RPM I-1640 + allitol | D-glucose | 2.4 | allitol | Undetectable |

| | | | | |
|---|---|---|---|---|
| Sugar content in disrupted cell suspension of J82 | | | | |

As a result of the analysis of the disrupted cell suspension of the bladder carcinoma cell line J82 by HPLC, the presence of D-glucose was found in the cells in all the media and its content was high in the cells cultured in the media RPMI-1640 + D-glucose and RPMI-1640 + D-allose, each having a high D-glucose content.

The cells cultured in RPMI-1640 + L-allulose contained 13.2 to 14.1 µg of L-allulose; the cells cultured in RPMI-1640 + D-allulose contained 11.8 to 14.3 µg of D-allulose; and the cells cultured in RPMI-1640 + D-fructose contained 11.1 to 15.0 µg of D-fructose.

**[Table 4]**

| Medium | Sugar | Content in cell (µg) | Sugar | Content in cell (µg) |
|---|---|---|---|---|
| RPM I-1640 | D-glucose | 14.2 | | |
| RPM I-1640 + D-glucose | D-glucose | 6.5 | | |
| RPM I-1640 + L-allulose | D-glucose | 10.2 | L-allulose | 16.4- 19.7 |
| RPM I-1640 + D-allulose | D-glucose | 7.5 | D-allulose | 14.6- 17.8 |
| RPM I-1640 + D-fructose | D-glucose | 4.1 | D-fructose | 0.0 |
| RPM I-1640 + D-allose | D-glucose | 11.2 | D-allose | 0.0 |
| RPM I-1640 + allitol | D-glucose | 9.2 | allitol | Undetectable |

| | | | | |
|---|---|---|---|---|
| Sugar content in disrupted cell suspension of LNCa | | | | |

As a result of the analysis of the disrupted cell suspension of the prostate carcinoma cell line LNCa by HPLC, the presence of D-glucose was found in the cells in all the media and its content was high in the cells cultured in the media RPMI-1640, RPMI-1640 + L-allulose, and RPMI-1640 + D-allose.

The cells cultured in RPMI-1640 + L-allulose contained 16.4 to 19.7 µg of L-allulose; and the cells cultured in RPMI-1640 + D-allulose contained 14.6 to 17.8 µg of D-allulose.

**[Table 5]**

| Medium | Sugar | Content in cell (µg) | Sugar | Content in cell (µg) |
|---|---|---|---|---|
| RPM I-1640 | D-glucose | 1.3 | | |
| RPM I-1640 + D-glucose | D-glucose | 4.4 | | |
| RPM I-1640 + L-allulose | D-glucose | 2.5 | L-allulose | 10.8-11.7 |
| RPM I-1640 + D-allulose | D-glucose | 3.9 | D-allulose | 12.6-15.5 |
| RPM I-1640 + D-fructose | D-glucose | 4.2 | D-fructose | 12.9-17.0 |
| RPM I-1640 + D-allose | D-glucose | 16.2 | D-allose | 0.0 |
| RPM I-1640 + allitol | D-glucose | 4.5 | allitol | Undetectable |

| | | | | |
|---|---|---|---|---|
| Sugar content in disrupted cell suspension of Du145 | | | | |

As a result of the analysis of the disrupted cell suspension of the prostate carcinoma cell line Du145 by HPLC, the presence of D-glucose was found in the cells in all the media and its content was high in the cells cultured in the medium RPMI-1640 + D-allose.

The cells cultured in RPMI-1640 + L-allulose contained 10.8 to 11.7 µg of L-allulose; the cells cultured in RPMI-1640 + D-allulose contained 12.6 to 15.5 µg of D-allulose; and the cells cultured in RPMI-1640 + D-fructose contained 12.9 to 17.0 µg of D-fructose.

**[Table 6]**

| Medium | Sugar | Content in cell (µg) | Sugar | Content in cell (µg) |
|---|---|---|---|---|
| RPM I-1640 | D-glucose | 2.2 | | |
| RPM I-1640 + D-glucose | D-glucose | 6.7 | | |
| RPM I-1640 + L-allulose | D-glucose | 1.5 | L-allulose | 17.9-21.6 |
| RPM I-1640 + D-allulose | D-glucose | 1.6 | D-allulose | 14.7-19.7 |
| RPM I-1640 + D-fructose | D-glucose | 2.7 | D-fructose | 16.5-20.1 |
| RPM I-1640 + D-allose | D-glucose | 3.8 | D-allose | 7.5 |
| RPM I-1640 + allitol | D-glucose | 2.1 | allitol | Undetectable |

| | | | | |
|---|---|---|---|---|
| Sugar content in disrupted cell suspension of PC-3 | | | | |

As a result of the analysis of the disrupted cell suspension of the prostate carcinoma cell line PC-3 by HPLC, the presence of D-glucose was found in the cells in all the media and its content was high in the cells cultured in the medium RPMI-1640 + D-glucose.

The cells cultured in RPMI-1640 + L-allulose contained 17.9 to 21.6 µg of L-allulose; the cells cultured in RPMI-1640 + D-allulose contained 14.7 to 19.7 µg of D-allulose; and the cells cultured in RPMI-1640 + D-fructose contained 16.5 to 20.1 µg of D-fructose.

In addition, the cells cultured in RPMI-1640 + D-allose contained 7.5 µg of D-allose.

**[Table 7]**

| Medium | Sugar | Content in cell (µg) | Sugar | Content in cell (µg) |
|---|---|---|---|---|
| RPM I-1640 | D-glucose | 2.3 | | |
| RPM I-1640 + D-glucose | D-glucose | 5.6 | | |
| RPM I-1640 + L-allulose | D-glucose | 2.1 | L-allulose | 16.6-23.4 |
| RPM I-1640 + D-allulose | D-glucose | 1.9 | D-allulose | 15.8-22.1 |
| RPM I-1640 + D-fructose | D-glucose | 2.1 | D-fructose | 16.8-19.0 |
| RPM I-1640 + D-allose | D-glucose | 1.8 | D-allose | 11.2 |
| RPM I-1640 + allitol | D-glucose | 1.8 | allitol | Undetectable |

| | | | | |
|---|---|---|---|---|
| Sugar content in disrupted cell suspension of ACHN | | | | |

As a result of the analysis of the disrupted cell suspension of the renal cell carcinoma cell line ACHN by HPLC, the presence of D-glucose was found in the cells in all the media and its content was high in the cells cultured in the medium RPMI-1640 + D-glucose.

The cells cultured in RPMI-1640 + L-allulose contained 16.6 to 23.4 µg of L-allulose; the cells cultured in RPMI-1640 + D-allulose contained 15.8 to 22.1 µg of D-allulose; and the cells cultured in RPMI-1640 + D-fructose contained 16.8 to 19.0 µg of D-fructose.

In addition, the cells cultured in RPMI-1640 + D-allose contained 11.2 µg of D-allose.

**[Table 8]**

| Medium | Sugar | Content in cell (µg) | Sugar | Content in cell (µg) |
|---|---|---|---|---|
| RPM I-1640 | D-glucose | 1.9 | | |
| RPM I-1640 + D-glucose | D-glucose | 4.4 | | |
| RPM I-1640 + L-allulose | D-glucose | 2.3 | L-allulose | 16.0-21.2 |
| RPM I-1640 + D-allulose | D-glucose | 2.3 | D-allulose | 17.2-23.1 |
| RPM I-1640 + D-fructose | D-glucose | 2.5 | D-fructose | 16.7-20.7 |
| RPM I-1640 + D-allose | D-glucose | 2.1 | D-allose | 13.2 |
| RPM 1-1640 + allitol | D-glucose | 2.4 | allitol | Undetectable |

| | | | | |
|---|---|---|---|---|
| Sugar content in disrupted cell suspension of Caki-I | | | | |

As a result of the analysis of the disrupted cell suspension of the renal cell carcinoma cell line Caki-I by HPLC, the presence of D-glucose was found in the cells in all the media and its content was high in the cells cultured in the medium RPMI-1640 + D-glucose.

The cells cultured in RPMI-1640 + L-allulose contained 16.0 to 21.2 µg of L-allulose; the cells cultured in RPMI-1640 + D-allulose contained 17.2 to 23.1 µg of D-allulose; and the cells cultured in RPMI-1640 + D-fructose contained 16.7 to 20.7 µg of D-fructose.

In addition, the cells cultured in RPMI-1640 + D-allose contained 13.2 µg of D-allose.

**[Table 9]**

| Medium | Sugar | Content in cell (µg) | Sugar | Content in cell (µg) |
|---|---|---|---|---|
| RPM I-1640 | D-glucose | 3.8 | | |
| RPM I-1640 + D-glucose | D-glucose | 8.4 | | |
| RPM I-1640 + L-allulose | D-glucose | 1.7 | L-allulose | 14.5-17.8 |
| RPM I-1640 + D-allulose | D-glucose | 3.1 | D-allulose | 16.5-21.4 |
| RPM I-1640 + D-fructose | D-glucose | 3.6 | D-fructose | 20.4-21.3 |
| RPM I-1640 + D-allose | D-glucose | 3.4 | D-allose | 11.2 |
| RPM I-1640 + allitol | D-glucose | 4.3 | allitol | Undetectable |

| | | | | |
|---|---|---|---|---|
| Sugar content in disrupted cell suspension of Caki-II | | | | |

As a result of the analysis of the disrupted cell suspension of the renal cell carcinoma cell line Caki-II by HPLC, the presence of D-glucose was found in the cells in all the media and its content was high in the cells cultured in the medium RPMI-1640 + D-glucose.

The cells cultured in RPMI-1640 + L-allulose contained 14.5 to17.8 µg of L-allulose; the cells cultured in RPMI-1640 + D-allulose contained 16.5 to 21.4 µg of D-allulose; and the cells cultured in RPMI-1640 + D-fructose contained 20.4 to 21.3 µg of D-fructose.

In addition, the cells cultured in RPMI-1640 + D-allose contained 11.2 µg of D-allose.

### <Summary of experimental results>

The uptake of D-allulose and L-allulose into the cells was found in all the human carcinoma cell lines. With respect to D-allose, on the other hand, no uptake was found in the human bladder carcinoma cell line. The uptake of D-allose was found only in one (PC-3) of three human prostate carcinoma cell lines, while the uptake into the cells was found in all the human renal cell carcinoma cell lines (ACHN, Caki-I, and Caki-II).

### Example 2

### [Analysis of antitumor effect of rare sugars with human renal cell carcinoma cell line as obj ect]

The antitumor effect of rare sugars was analyzed using the three human renal cell carcinoma cell lines (ACHN, Caki-I, and Caki-II) used in Example 1. As the rare sugars, used were the following ten rare sugars: L-allulose, D-allulose, D-allose, L-fructose, D-mannose, L-sorbose, D-tagatose, D-galactose, D-sorbose, and L-tagatose, while as monosaccharides not belonging to rare sugars, D-glucose and D-fructose were used.

### [Medium used]

As a medium, a minimum essential medium (MEM) containing 1000 mg/l of D-glucose was used. By adjusting it to contain 10 mM, 25 mM, and 50 mM of each of the monosaccharides or rare sugars and using thus-obtained MEMs [shown by (10), (25), and (50) in the drawing], an antitumor effect of the rare sugars was evaluated. In control, MEM did not contain a sugar.

### [Procedure of experiment]

A 5.0 × 10⁴ cells/ml of each of cell suspensions was formed using MEM. The resulting suspension (0.1 ml/well) was distributed in a 96-well plate, followed by culturing for 24 hours. Then, the culture solution was removed and 0.1 ml/well of each of culture solutions containing 10 mM, 25 mM, or 50 mM of the monosaccharide or rare sugar was added to the MEM and the resulting mixture was cultured for 24 hours (#1 to #13).
- #1:: MEM solution alone (Control, in the figure)
- #2:: D-glucose solution (D-Glucose, in the figure)
- #3:: L-allulose solution (L-Allulose, in the figure)
- #4:: D-allulose solution (D-Allulose in the figure)
- #5:: D-fructose solution (D-Fructose, in the figure)
- #6:: D-allose solution (D-Allose, in the figure)
- #7:: L-fructose solution (L-Fructose, in the figure)
- #8:: D-mannose solution (D-Mannose, in the figure)
- #9:: L-sorbose solution (L-Sorbose, in the figure)
- #10:: D-tagatose solution (D-Tagatose, in the figure)
- #11:: D-galactose solution (D-Galactose, in the figure)
- #12:: D-sorbose solution (D-Sorbose, in the figure)
- #13:: L-tagatose solution (L-Tagatose, in the figure)

### [Cell survival assay (MTT assay)]

The MTT assay is one of test methods for measuring survival and makes use of a color reaction of an insoluble formazan dye (blue) caused by the reduction of MTT which is a tetrazolium salt. The reduction of MTT occurs by succinate-tetrazolium reductase which is a reductase of mitochondria. This enzymatic activity is high and a color reaction is recognized in living cells but the color reaction fades away due to cell death including apoptosis. The cell viability is determined by measuring this color reaction with a microplate reader.

### [Results of cell survival assay]

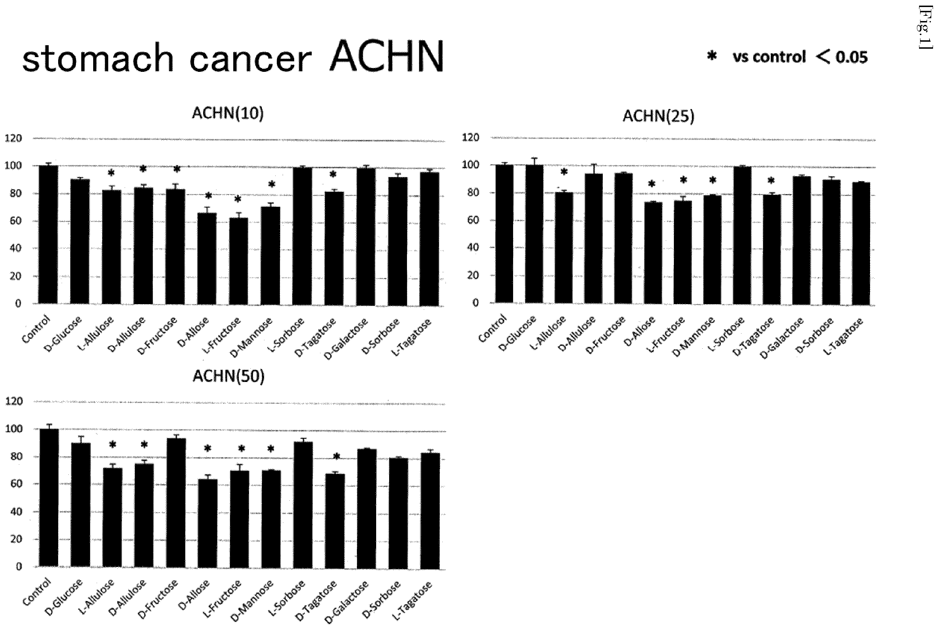

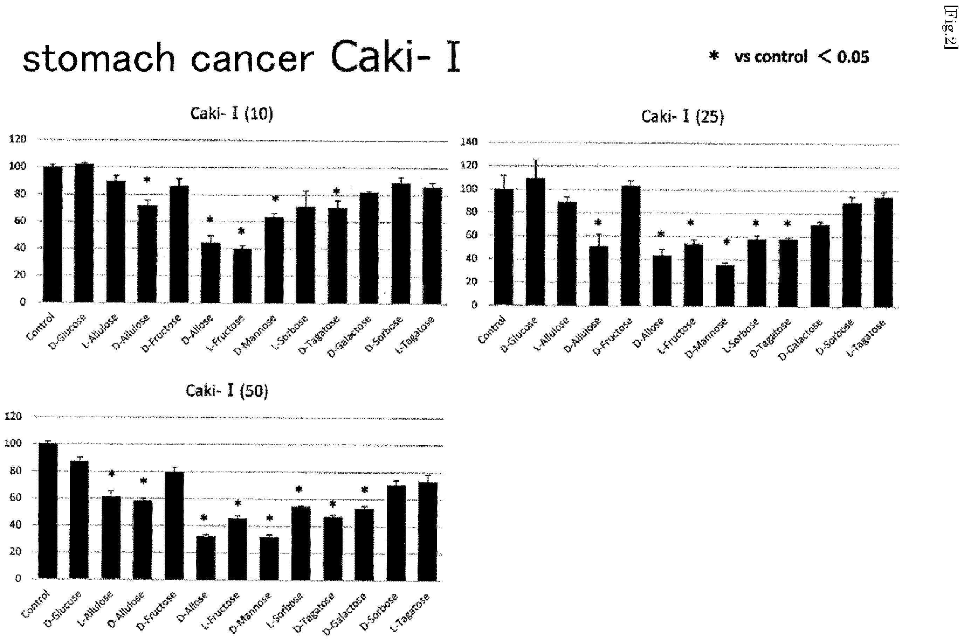

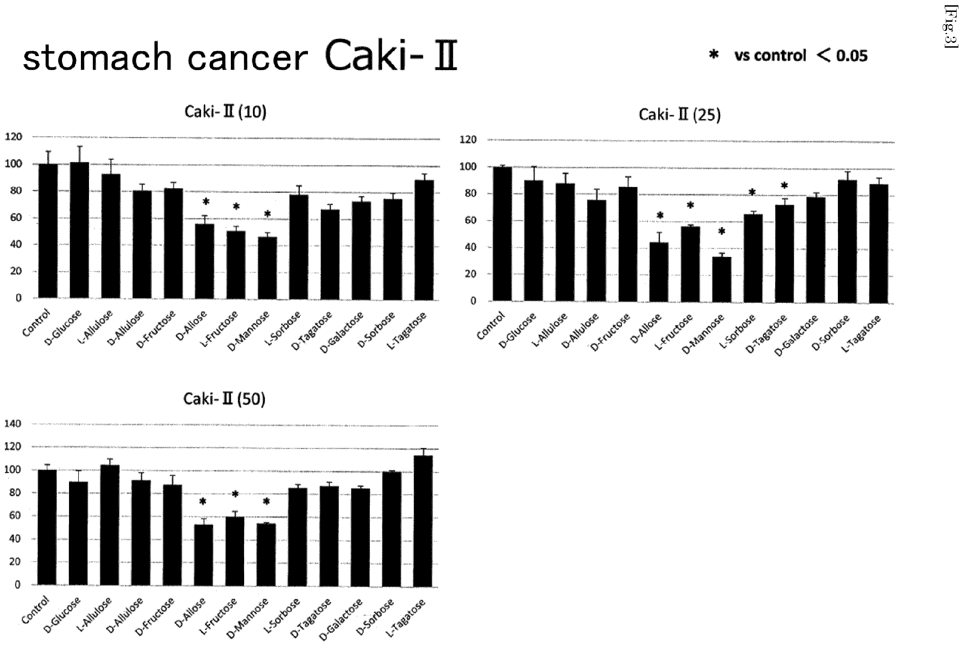

The measurement results of the viability of human renal cell carcinoma cells are shown in Fig. 1 to Fig. 3. Fig. 1 shows the results of the survival assay of the renal cell carcinoma cell line (ACHN), Fig. 2 shows the results of the survival assay of the renal cell carcinoma cell line (Caki-I), and Fig. 3 shows the results of survival assay of the renal cell carcinoma cell line (Caki-II). As shown in Fig. 1 to Fig. 3, D-allose significantly reduced the viability at any of the concentrations 10 mM, 25 mM, and 50 mM in the above three cell lines compared with Control (p<0.05) and exhibited the strongest antitumor effect among the analyzed sugars.

### [Consideration]

In carcinoma cells, ATP production in mitochondria is suppressed. In carcinoma cells, a metabolic system called "glycolysis" which produces ATP from glucose without using oxygen is accelerated. Glycolysis occurs in the cytoplasm. There are several reasons why carcinoma cells suppress aerobic respiration in mitochondria.

One of them is because a large amount of glucose is necessary as a material for synthesizing cell constituents. For increasing the number of cells by cell division, cell constituents such as nucleic acids, cell membranes, and proteins should be formed newly. Cells can produce nucleic acids, lipids, and amino acids through a glycolytic pathway or various intracellular metabolic pathways derived therefrom. When oxygen is used in mitochondria and glucose is used completely for ATP production, there are no more materials for forming cells.

In addition, aerobic respiration in mitochondria increases the production of active oxygen. The active oxygen gives a damage to cells, suppresses proliferation or metastasis, and becomes a cause of cell death. Carcinoma cells are presumed to suppress the use of oxygen in mitochondria to prevent an increase in the production of active oxygen. It is convenient for the survival or growth of carcinoma cells to suppress the metabolism that uses oxygen in mitochondria.

In the case of carcinoma cells, it has been known that the growth or metastasis is suppressed and cell death is caused by enhancing the activity of mitochondria in carcinoma cells. This is because materials for increasing the number of cells become insufficient due to complete glycolysis, aerobic respiration is accelerated to increase the production of active oxygen, and the damage caused by the active oxygen leads to the self-destruction of carcinoma cells. In short, a treatment method by activating the mitochondria in cells is presumed to kill only carcinoma cells while enhancing the function of normal cells.

It has been suggested that D-allose taken up specifically into human renal cell carcinoma cells enhances mitochondrial activity and thereby exhibits an antitumor effect

### Example 3

### [Method of preparing renal cell carcinoma xenograft mouse]

The experiment of the present example used two human renal cell carcinoma cell lines (Caki-I and ACHN). Each cell was cultured under the wet environment of 37°C and 5% CO₂ by using a RPMI-1640 culture solution (2000 mg D-glucose/L) containing a 10% fetal calf serum, a HEPES buffer, and penicillin-streptomycin.

With respect to a renal cell carcinoma xenograft mouse model, a 1.0 × 10⁵ cells/ml of a cell suspension was obtained by adjusting the cultured cells with a MEM culture solution and then 0.1 ml of the resulting suspension was injected into the femoral subcutaneous tissue of Female athymic nude mice (BALB/c-nu/nu, 6-week-old). After checking that the tumor volume was 200 mm³ or more one week after the injection, the mice were provided for the experiment thereafter.

### [Changes of D-allose concentration after intraperitoneal injection of D-allose to renal cell carcinoma xenograft mouse model]

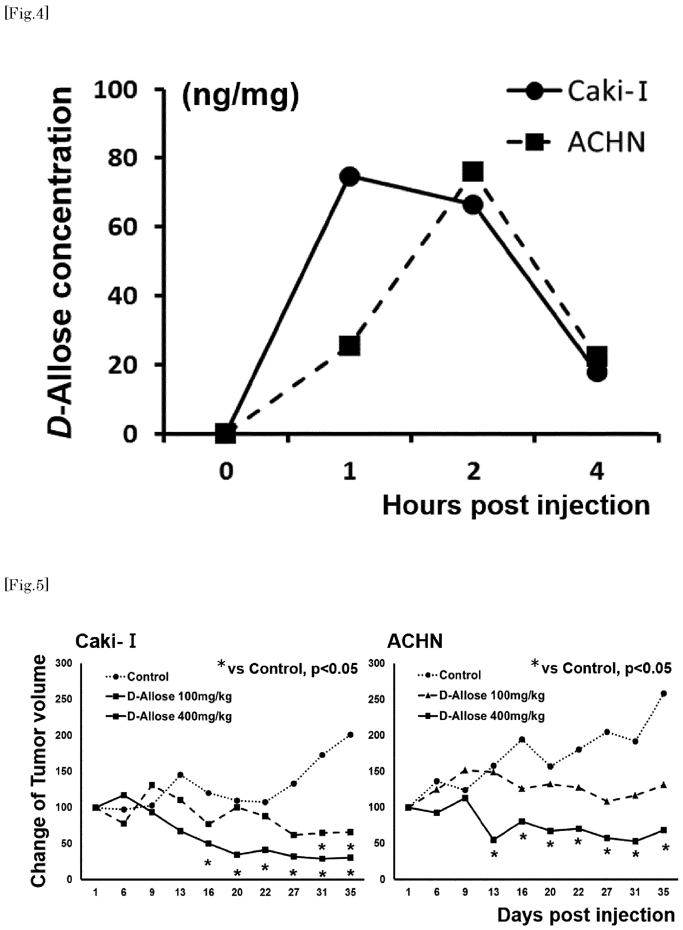

A 400 mg/kg/0.4 mL solution of rare sugar D-allose was prepared using physiological saline and was intraperitoneally injected into the xenograft mouse models of two human renal cell carcinoma cell lines (Caki-I and ACHN) through a 27G needle. A tumor was enucleated from the mouse before D-allulose administration and 1 hour, 2 hours, and 4 hours after the administration. The tumor was ultrasonically disrupted in 1 mL of PBS, followed by centrifugal separation for 5 minutes at 3000 revolutions. A monosaccharide in the supernatant thus obtained was labeled with ABEE (4-aminobenzoic acid ethyl ester) and the quantitative analysis of D-allose was performed by HPLC (high performance liquid chromatography). Fig. 4 shows the change of a D-allose concentration in the tumor of the renal cell carcinoma xenograft mouse models after intraperitoneal D-allose injection thereinto. As shown in Fig. 4, one hour after the intraperitoneal D-allose injection into the renal cell carcinoma xenograft mouse models, D-allose was detected from both the Caki-I and ACHN tumors. The D-allose concentration became the highest in Caki-I one hour after the intraperitoneal injection and in ACHN two hours after the injection. The D-allose concentration in the tumor decreased thereafter.

### [Changes of tumor volume in renal cell carcinoma xenograft mouse model by intraperitoneal D-allose injection]

The day when the tumor size of the renal cell carcinoma xenograft mouse models became 200 mm³ or more was called day 0 and the intraperitoneal injection of a D-allose-containing solution was started from day 1. The solution injected is 0.4 mL of physiological saline or a solution obtained by dissolving 100 mg/kg or 400 mg/kg of D-allose in 0.4 mL physiological saline. The resulting solution was intraperitoneally injected into three groups of mice (Control, 100 mg/kg of D-allose, and 400 mg/kg of D-allose) once a day for 5weeks in total. The body weight of the mice and the long and short diameters of the tumor were measured over time twice a week. It is to be noted that the tumor volume was obtained as a product of long diameter × short diameter × short diameter of the tumor × 0.5. On day 35 after the intraperitoneal injection was started, the tumor as well as the liver and kidney were excised from the mice and provided for the experiment performed thereafter.

The change of a tumor volume in the renal cell carcinoma xenograft mouse models caused by the intraperitoneal D-allose injection is shown in Fig. 5.

As shown in Fig. 5, in the tumor volume evaluation of the xenograft mouse model made using ACHN, the tumor volume of the 400 mg/kg D-allose group was significantly smaller than the Control group on and after day 13. In the xenograft mouse model made using Caki-I, the tumor volume on and after day 16 in the 400 mg/kg D-allose group and the tumor volume on and after day 31 in the 100 mg/kg D-allose group were significantly smaller than that of the Control group; and in addition, the tumor volume on and after day 16 in the 400 mg/kg D-allose group was significantly smaller than the tumor volume before administration. The above results show that not only a tumor growth suppressing effect but also a tumor size reducing effect, though depending on the administration amount of D-allose, can be expected (Mann-Whitney U test).

### [Anti-tumor effect of D-allose in renal cell carcinoma xenograft mouse model: necrosis area and nuclear fission]

The tissue of the excised tumor, kidney, and liver was fixed with a 4% paraformaldehyde phosphate buffer, embedded with paraffin, and sliced into a 4-µm thick section. The resulting sliced section was stained with hematoxylin and eosin and evaluated by a medical specialist in pathology. The influence of D-allose on the tumor tissue was evaluated by a necrosis area (%) and an image of the number of nuclear fissions (Cells/HPF). It is to be noted that the necrosis area was measured using an image recording software Nikon NIS-Elements D and figures after the decimal point were omitted. On the other hand, the number of nuclear fissions was obtained as an average of those of three fields of view and figures after the decimal point were also omitted. The anti-tumor effect of D-allose, more specifically, necrosis area and the number of nuclear fissions in the renal cell carcinoma xenograft mouse model are shown in Table 10.

**[Table 10]**

| | Caki- I | | | ACHN | |
|---|---|---|---|---|---|
| | Necrosis area (%) | Nuclear fission (Cells/HPF) | | Necrosis area (%) | Nuclear fission (Cells/HPF) |
| Control | 11 | 1.2 | | 16 | 3.1 |
| D-allose 100mg/kg | 22 | 0.9 | | 49 | 1.2 |
| D-allose 400mg/kg | 33 | 1 | | 69 | 0.9 |

As a result, in tumor tissue of the renal cell carcinoma xenograft mouse models made using Caki-I and ACHN, the necrosis area increased and the number of nuclear fissions decreased, depending on the D-allose administration amount.

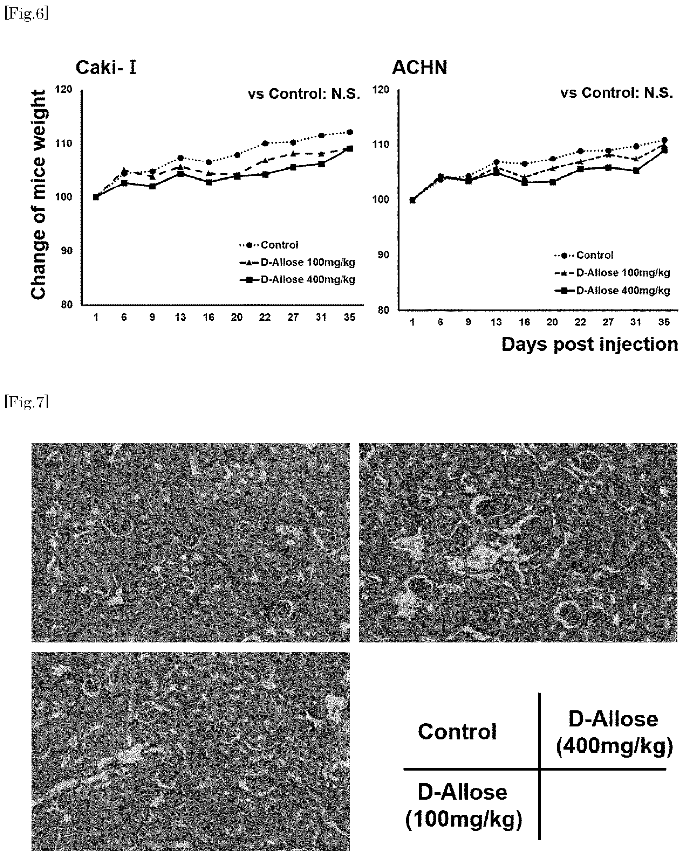

Fig. 6 shows a graph of the changes of a body weight as the influence of D-allose on the renal cell carcinoma xenograft mouse models. No significant difference in mice weight was recognized among three groups (Mann-Whitney U test).

Fig. 7 shows a micrograph (×100) showing the change in the kidney tissue as the influence of D-allose in the renal cell carcinoma xenograft mouse models. No influence of D-allose on the kidney tissue is recognized (diagnosis by a medical specialist in pathology).

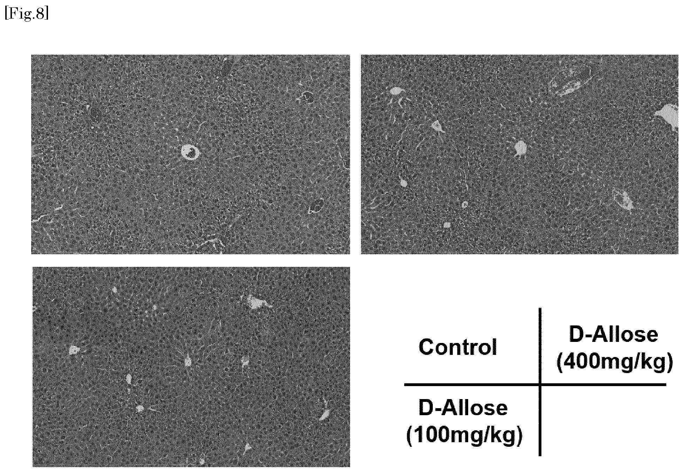

Fig. 8 shows a micrograph (×100) showing the change in the liver tissue as the influence of D-allose in the renal cell carcinoma xenograft mouse models. No influence of D-allose on the liver tissue is recognized (diagnosis by a medical specialist in pathology).

### Industrial Applicability

The present inventors have found that D-allose is taken up specifically into the human renal cell carcinoma cells among human carcinoma cells and D-allose thus taken up has antitumor activity. D-Allose is water soluble so that it has high membrane permeability and moreover, it does not form an aggregate because of solubility in an aqueous medium.

In addition, since it is taken up specifically into renal cell carcinoma cells, it can be used as a drug carrier capable of delivering an anticancer agent selectively to renal cell carcinoma cells, can enhance the uptake of the anticancer agent into the renal cell carcinoma cells, and can make both the D-allose and the anticancer agent thus taken up perform their antitumor activity. It is therefore expected as a breakthrough molecular targeted remedy for renal cell carcinoma.

## Claims

1. An agent, comprising D-allose and making use of a property of being taken up selectively into renal cell carcinoma cells.

2. The agent according to Claim 1, wherein D-allose taken up into renal cell carcinoma cells exhibits an antitumor effect.

3. The agent according to Claim 1 or 2, wherein D-allose is D-allose and/or a derivative thereof, and/or a mixture thereof.

4. The agent according to Claim 3, wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.

5. A selective drug delivery carrier to renal cell carcinoma cells, comprising D-allose.

6. The selective drug delivery carrier to renal cell carcinoma cells according to Claim 5, wherein D-allose taken up into renal cell carcinoma cells exhibits an antitumor effect.

7. The selective drug delivery carrier to renal cell carcinoma cells according to Claim 4 or 5, wherein D-allose is D-allose and/or a derivative thereof, and/or a mixture thereof.

8. The selective drug delivery carrier to renal cell carcinoma cells according to Claim 7, wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.

9. The selective drug delivery carrier to renal cell carcinoma cells according to any of Claims 5 to 8, wherein the drug contains an anticancer agent.

10. The selective drug delivery carrier to renal cell carcinoma cells according to any of Claims 5 to 9, wherein D-allose and the drug are associated through covalent bonding directly or via a linker.

11. The selective drug delivery carrier to renal cell carcinoma cells according to any of Claims 5 to 10, wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

12. A composition for the treatment of renal cell carcinoma, comprising D-allose.

13. The composition for the treatment of renal cell carcinoma according to Claim 12, wherein D-allose taken up into renal cell carcinoma cells exhibits an antitumor effect.

14. The composition for the treatment of renal cell carcinoma according to Claim 12 or 13, wherein D-allose is D-allose and/or a derivative thereof, and/or a mixture thereof.

15. The composition for the treatment of renal cell carcinoma according to Claim 14, wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.

16. The composition for the treatment of renal cell carcinoma according to any of Claims 12 to 15, further containing a drug associated with D-allose.

17. The composition for the treatment of renal cell carcinoma according to Claim 16, wherein the drug contains an anticancer agent.

18. The composition for the treatment of renal cell carcinoma according to Claim 16 or 17, wherein D-allose and the drug are associated through covalent bonding directly or via a linker.

19. The composition for the treatment of renal cell carcinoma according to any of Claims 16 to 18, wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

20. A drug delivery method to renal cell carcinoma cells, comprising supporting a drug on a drug delivery carrier to obtain a medicament, using the medicament for renal cell carcinoma cells, allowing the medicament to be taken up selectively into the renal cell carcinoma cells, and thereby delivering the drug selectively to the cells, wherein the carrier is D-allose and the medicament is obtained by supporting the drug on the D-allose through chemical bonding.

21. The drug delivery method according to Claim 20, which is a method making use of a property of D-allose being taken up into carcinoma cells and enhancing the uptake of the drug into the renal cell carcinoma cells.

22. The drug delivery method according to Claim 20 or 21, wherein the carrier is D-allose and the medicament is obtained by supporting the drug on the D-allose by associating therewith through covalent bonding directly or via a linker.

23. The drug delivery method according to any of Claims 20 to 22, wherein the drug contains an anticancer agent.

24. The drug delivery method according to any of Claims 20 to 23, wherein the drug is a radioactive isotope, an enzyme, a prodrug activating enzyme, a radiosensitizer, an iRNA, an alkylating agent, a purine antagonist, a pyrimidine antagonist, a plant alkaloid, an intercalating antibiotic, an antimetabolite, an aromatase inhibitor, a mitotic inhibitor, a growth factor inhibitor, a cell cycle inhibitor, or a topoisomerase inhibitor.

25. The drug delivery method according to any of Claims 20 to 24, wherein D-allose is D-allose and/or a derivative thereof, and/or a mixture thereof.

26. The drug delivery method according to Claim 25, wherein the derivative of D-allose is a D-allose derivative selected from sugar alcohols obtained by converting the carbonyl group of D-allose into an alcohol group, uronic acids obtained by oxidizing the alcohol group of D-allose, and amino sugars obtained by substituting the alcohol group of D-allose by an NH₂ group.
